# EUROPEAN PATENT APPLICATION

(11) **EP 3 730 143 A1**
(43) Date of publication of application: **28.10.2020**
(21) Application number: 18890141.7
(22) Date of filing: 20.12.2018
(51) Int. Cl.: A61K 31/551, A61K 31/444, A61K 31/472, A61P 27/02, A61P 27/06, A61P 43/00

(54) **OMIDENEPAG COMBINATION**

(30) Priority: 21.12.2017 JP 2017244846; 26.09.2018 JP 2018180657
(71) Applicant: Santen Pharmaceutical Co., Ltd., Kita-ku Osaka-shi Osaka 530-8552 (JP)
(72) Inventor: FUWA Masahiro, Ikoma-shi, Nara 630-0101 (JP); TANIGUCHI Takazumi, Ikoma-shi, Nara 630-0101 (JP)
(74) Representative: Müller-Boré & Partner Patentanwälte PartG mbB
(86) International application number: PCT/JP2018/046969
(87) International publication number: WO 2019/124487

(57) **Abstract**

An object of the present invention is to find out a combination of a prophylactic or therapeutic agent for glaucoma or ocular hypertension, which is useful as a prophylactic or therapeutic agent for glaucoma or ocular hypertension.

By combining omidenepag and ripasudil or netarsudil, each complement and/or enhances the intraocular pressure lowering effect. As the form of administration, they may be administered concomitantly or may be administered as a combination drug.

## Description

### TECHNICAL FIELD

The present invention relates to a prophylactic or therapeutic agent for glaucoma or ocular hypertension, which is characterized in that omidenepag or an ester thereof or a salt thereof, and one or more of the Rho-associated coiled-coil containing protein kinase inhibitors selected from the group consisting of ripasudil, netarsudil and a salt thereof are administered in combination. The present invention also relates to a prophylactic or therapeutic agent for glaucoma or ocular hypertension comprising omidenepag or an ester thereof or a salt thereof, which is characterized by being used concomitantly with one or more of the Rho-associated coiled-coil containing protein kinase inhibitors selected from the group consisting of ripasudil, netarsudil and a salt thereof.

### BACKGROUND ART

Glaucoma is a refractory eye disease caused by suffering from damage of the internal tissue (retina, optic nerve, etc.) of the eyeball due to the intraocular pressure increase resulted from various pathogenesis. As a method for treating glaucoma, intraocular pressure lowering therapy is generally used, and typical examples thereof include drug therapy, laser therapy, surgical therapy, etc.

In the drug therapy, drugs such as sympathomimetics (non-selective stimulants such as dipivefrin, etc., and α₂ receptor agonists such as brimonidine, etc.), sympathetic nerve blockers (β receptor blockers such as timolol, befunolol, carteolol, nipradilol, betaxolol, levobunolol, metipranolol, etc., and α₁ receptor blockers such as bunazosin hydrochloride, etc.), parasympathomimetics (pilocarpine, etc.), carbonic anhydrase inhibitors (acetazolamide, etc.), prostaglandins (isopropyl unoprostone, latanoprost, travoprost, bimatoprost, etc.), and Rho-associated coiled-coil containing protein kinase inhibitors (ripasudil), etc., have been used.

Also, in order to obtain a more potent effect of lowering an intraocular pressure, some reports have been made that drugs having an intraocular pressure lowering action are used in combination. For example, in JP Patent No. 2,726,672 (Patent Document 2), administration of a combination of a sympathetic nerve blocker and a prostaglandin has been reported. Also, in WO 2002/38158 (Patent Document 3), a therapeutic method for glaucoma by administering several drugs having an intraocular pressure lowering effect in combination to the eye has been disclosed. Further, in WO 2004/019951 (Patent Document 4), administration of a combination of a Rho-associated coiled-coil containing protein kinase inhibitor and a prostaglandin has been reported, and in WO 2004/045644 (Patent Document 5), combination administration of a Rho-associated coiled-coil containing protein kinase inhibitor and a β receptor blocker has been reported. In addition, a combination drug of dorzolamide and timolol, a combination drug of latanoprost and timolol, a combination drug of brimonidine and timolol and the like are commercially available (Non-Patent Document 1).

By the way, omidenepag is a compound as one of a vast number of pyridylaminoacetic acid compounds described in Patent Document 6 and Patent Document 7. Since these pyridylaminoacetic acid compounds have an EP2 agonistic action, there are described that they are expected to exhibit an intraocular pressure lowering action and can be a therapeutic agent for glaucoma.

Further, in Patent Document 8, there is described that omidenepag exhibits a particularly excellent intraocular pressure lowering action when it is contained at a specific content, and in Patent Document 9, there is described that omidenepag is useful as a therapeutic agent for diseases accompanied by highly elevated intraocular pressure. Also, in Patent Documents 10 to 12, there is described a specific formulation containing omidenepag as an active ingredient.

In Patent Documents 13 and 14, there are described that an intraocular pressure lowering action is enhanced by using omidenepag in combination with other therapeutic agents for glaucoma such as timolol or the like, and there is a description about a combination of omidenepag and a Rho-associated coiled-coil containing protein kinase inhibitor. However, there is no specific description of ripasudil or netarsudil as a Rho-associated coiled-coil containing protein kinase inhibitor, and there is completely no description as to what effect is exhibited when omidenepag is used in combination with ripasudil or netarsudil.

### PRIOR ART DOCUMENTS

### [Patent Documents]

Patent Document 1: WO 2010/113957
Patent Document 2: JP Patent No. 2,726,672
Patent Document 3: WO 2002/38158
Patent Document 4: WO 2004/019951
Patent Document 5: WO 2004/045644
Patent Document 6: U.S. Patent Application Publication No. 2012/0190852
Patent Document 7: U.S. Patent Application Publication No. 2011/0054172
Patent Document 8: U.S. Patent Application Publication No. 2015/0196541
Patent Document 9: WO 2017/006985
Patent Document 10: U.S. Patent Application Publication No. 2016/0317512
Patent Document 11: U.S. Patent Application Publication No. 2016/0317664
Patent Document 12: WO 2017/002941
Patent Document 13: U.S. Patent Application Publication No. 2014/0018396
Patent Document 14: U.S. Patent Application Publication No. 2014/0018350

### [Non-Patent Documents]

Non-Patent Document 1: Clinical Ophthalmology, 2010, 4, 1-9

### SUMMARY OF THE INVENTION

### [Problems to be Solved by the Invention]

It is a very interesting task to find out a novel combination of prophylactic or therapeutic agents for glaucoma or ocular hypertension, which is useful as a prophylactic or therapeutic agent for glaucoma or ocular hypertension.

### [Means for Solving the Problems]

The present inventors have intensively studied the effect of the combination of prophylactic or therapeutic agents for glaucoma or ocular hypertension, and as a result, they have found that by using omidenepag and ripasudil or netarsudil in combination, an intraocular pressure lowering action is enhanced as compared with the case where each drug is used alone, whereby they have accomplished the present invention.

That is, the present invention relates to the following.
(1) A prophylactic or therapeutic agent for glaucoma or ocular hypertension, which is characterized in that omidenepag or an ester thereof or a salt thereof, and one or more of the Rho-associated coiled-coil containing protein kinase inhibitors selected from the group consisting of ripasudil, netarsudil and a salt thereof are administered in combination.
(2) The prophylactic or therapeutic agent described in the above-mentioned (1), which is a combination drug comprising omidenepag or an ester thereof or a salt thereof, and one or more of the Rho-associated coiled-coil containing protein kinase inhibitors selected from the group consisting of ripasudil, netarsudil and a salt thereof.
(3) The prophylactic or therapeutic agent described in the above-mentioned (1), wherein omidenepag or an ester thereof or a salt thereof, and one or more of the Rho-associated coiled-coil containing protein kinase inhibitors selected from the group consisting of ripasudil, netarsudil and a salt thereof are administered at different times or simultaneously.
(4) A prophylactic or therapeutic agent for glaucoma or ocular hypertension comprising omidenepag or an ester thereof or a salt thereof, which is characterized by being used concomitantly with one or more of the Rho-associated coiled-coil containing protein kinase inhibitors selected from the group consisting of ripasudil, netarsudil and a salt thereof.
(5) The prophylactic or therapeutic agent described in the above-mentioned (4), which is administered at different time from or simultaneously with one or more of the Rho-associated coiled-coil containing protein kinase inhibitors selected from the group consisting of ripasudil, netarsudil and a salt thereof.
(6) The prophylactic or therapeutic agent described in any one of the above-mentioned (1) to (5), wherein omidenepag or an ester thereof or a salt thereof is omidenepag isopropyl.
(7) The prophylactic or therapeutic agent described in any one of the above-mentioned (1) to (6), wherein the Rho-associated coiled-coil containing protein kinase inhibitor is ripasudil monohydrochloride dihydrate.
(8) The prophylactic or therapeutic agent described in any one of the above-mentioned (1) to (6), wherein the Rho-associated coiled-coil containing protein kinase inhibitor is dimesylate or dihydrochloride of netarsudil.
   Also, the present invention relates to the following.
(9) An intraocular pressure-lowering agent, which is characterized in that omidenepag or an ester thereof or a salt thereof, and one or more of the Rho-associated coiled-coil containing protein kinase inhibitors selected from the group consisting of ripasudil, netarsudil and a salt thereof are combined.
(10) An intraocular pressure-lowering agent comprising omidenepag or an ester thereof or a salt thereof, which is characterized by being used concomitantly with one or more of the Rho-associated coiled-coil containing protein kinase inhibitors selected from the group consisting of ripasudil, netarsudil and a salt thereof.
   Further, the present invention relates to the following.
(11) A prophylactic or therapeutic composition for glaucoma or ocular hypertension comprising omidenepag or an ester thereof or a salt thereof, which is characterized by being administered in combination with one or more of the Rho-associated coiled-coil containing protein kinase inhibitors selected from the group consisting of ripasudil, netarsudil and a salt thereof.
(12) A prophylactic or therapeutic method for glaucoma or ocular hypertension comprising: administering a therapeutically effective amount of omidenepag or an ester thereof or a salt thereof, and a therapeutically effective amount of one or more the Rho-associated coiled-coil containing protein kinase inhibitors selected from the group consisting of ripasudil, netarsudil and a salt thereof, to a subject in need thereof.
(13) Use of a combination of omidenepag or an ester thereof or a salt thereof, and one or more of the Rho-associated coiled-coil containing protein kinase inhibitors selected from the group consisting of ripasudil, netarsudil and a salt thereof for manufacturing a medicament for the prophylaxis or treatment for glaucoma or ocular hypertension.
(14) Use of omidenepag or an ester thereof or a salt thereof for manufacturing a medicament for the prophylaxis or treatment for glaucoma or ocular hypertension characterized by being used concomitantly with one or more of the Rho-associated coiled-coil containing protein kinase inhibitors selected from the group consisting of ripasudil, netarsudil and a salt thereof.
(15) Omidenepag or an ester thereof or a salt thereof for use in the prophylaxis or treatment for glaucoma or ocular hypertension, which is characterized by being used concomitantly with one or more of the Rho-associated coiled-coil containing protein kinase inhibitors selected from the group consisting of ripasudil, netarsudil and a salt thereof.
(16) A combination of omidenepag or an ester thereof or a salt thereof, and one or more of the Rho-associated coiled-coil containing protein kinase inhibitors selected from the group consisting of ripasudil, netarsudil and a salt thereof for use in the prophylaxis or treatment for glaucoma or ocular hypertension.
   Moreover, the present invention relates to the following.
(17) A composition for lowering an intraocular pressure comprising omidenepag or an ester thereof or a salt thereof, which is characterized by being administered in combination with one or more of the Rho-associated coiled-coil containing protein kinase inhibitors selected from the group consisting of ripasudil, netarsudil and a salt thereof.
(18) A method for lowering an intraocular pressure comprising: administering a therapeutically effective amount of omidenepag or an ester thereof or a salt thereof, and a therapeutically effective amount of one or more of the Rho-associated coiled-coil containing protein kinase inhibitors selected from the group consisting of ripasudil, netarsudil and a salt thereof to a subject in need thereof.
(19) Use of a combination of omidenepag or an ester thereof or a salt thereof, and one or more of the Rho-associated coiled-coil containing protein kinase inhibitors selected from the group consisting of ripasudil, netarsudil and a salt thereof for manufacturing a medicament for lowering an intraocular pressure.
(20) Use of omidenepag or an ester thereof or a salt thereof for manufacturing a medicament for lowering an intraocular pressure characterized by being used concomitantly with one or more of the Rho-associated coiled-coil containing protein kinase inhibitors selected from the group consisting of ripasudil, netarsudil and a salt thereof.
(21) Omidenepag or an ester thereof or a salt thereof for use in lowering an intraocular pressure, which is characterized by being used concomitantly with one or more of the Rho-associated coiled-coil containing protein kinase inhibitors selected from the group consisting of ripasudil, netarsudil and a salt thereof.
(22) A combination of omidenepag or an ester thereof or a salt thereof, and one or more of the Rho-associated coiled-coil containing protein kinase inhibitors selected from the group consisting of ripasudil, netarsudil and a salt thereof for use in lowering an intraocular pressure.

Incidentally, each constitution of the above-mentioned (1) to (22) can be combined by arbitrary selecting two or more.

### [Effects of the Invention]

By administering omidenepag and ripasudil or netarsudil to an eye in combination, an intraocular pressure lowering action is enhanced. Accordingly, the present invention is useful as a prophylactic or therapeutic agent for glaucoma or ocular hypertension. Further, according to the present invention, sufficient safety as a pharmaceutical product is ensured.

### BRIEF DESCRIPTION OF THE DRAWINGS

FIG. 1 is a graph showing change in the lowering width of an intraocular pressure with the lapse of time for each administered group of omidenepag isopropyl and ripasudil alone, and concomitant use.
FIG. 2 is a graph showing change in the lowering width of an intraocular pressure with the lapse of time for each administered group of omidenepag isopropyl and netarsudil alone, and concomitant use.

### DESCRIPTION OF THE EMBODIMENTS

In the following, the present invention will be explained in detail.

The present invention is directed to a prophylactic or therapeutic agent for glaucoma or ocular hypertension, which is characterized in that omidenepag or an ester thereof or a salt thereof, and one or more of the Rho-associated coiled-coil containing protein kinase inhibitors selected from the group consisting of ripasudil, netarsudil and a salt thereof are administered in combination, and hereinafter, these are also simply referred to as the "therapeutic agent or the like".

In the therapeutic agent or the like of the present invention, omidenepag is the compound (CAS registry number: 1187451-41-7) represented by the following formula (1): 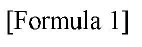 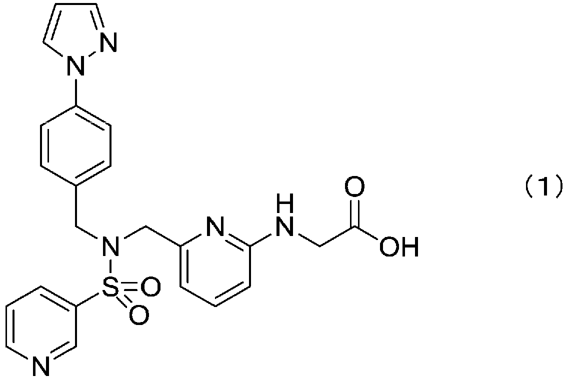 and is also referred to as (6-{[4-(pyrazol-1-yl)benzyl](pyridin-3-ylsulfonyl)aminomethyl}pyridin-2-ylamino)acetic acid.

In the therapeutic agent or the like of the present invention, an ester of omidenepag is preferably an ester formed by dehydration condensation of the carboxyl group of omidenepag with a monovalent alcohol having 1 to 6 carbon atoms, and more preferably an ester formed by dehydration condensation of the carboxyl group of omidenepag with a monovalent alcohol having 2 to 5 carbon atoms, further preferably 3 to 4 carbon atoms. As specific ester, example includes methyl ester, ethyl ester, n-propyl ester, isopropyl ester, n-butyl ester, isobutyl ester, sec-butyl ester, tert-butyl ester, n-pentyl ester or n-hexyl ester, preferably ethyl ester, n-propyl ester or isopropyl ester, and more preferably isopropyl ester. The isopropyl ester of omidenepag is specifically the compound (CAS registry number: 1187451-19-9) represented by the following formula (2): 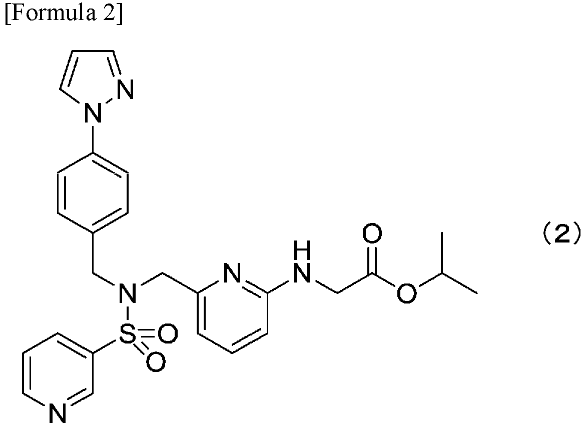 which is also referred to as omidenepag isopropyl or isopropyl (6-{[4-(pyrazol-1-yl)benzyl](pyridin-3-ylsulfonyl)aminomethyl}pyridin-2-ylamino)acetate. Since it has an EP2 agonistic action and exhibits an intraocular pressure lowering effect, it has been developed as a therapeutic agent for glaucoma and ocular hypertension.

In the therapeutic agent or the like of the present invention, the salt of omidenepag or the salt of omidenepag ester is not particularly limited as long as it is a pharmacologically acceptable salt. Specific examples include an inorganic acid salt such as hydrochloride, hydrobromide, hydroiodide, nitrate, sulfate or phosphate; an organic acid salt such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate or aspartate; a metal salt such as sodium salt, potassium salt, calcium salt or magnesium salt; an inorganic salt such as ammonium salt; or an organic amine salt such as triethylamine salt or guanidine salt, and preferably hydrochloride or trifluoroacetate.

In the therapeutic agent or the like of the present invention, omidenepag or an ester thereof or a salt thereof can be produced in accordance with the methods disclosed in U.S. Patent Application Publication No. 2012/0190852 (Patent Document 6), U.S. Patent Application Publication No. 2011/0054172 (Patent Document 7), U.S. Patent Application Publication No. 2017/0121288 or U.S. Patent Application Publication No. 2017/0114043, or a usual method in this technical field. Incidentally, the terms "omidenepag or an ester thereof or a salt thereof' used in the present application have means including (1) omidenepag , (2) an ester of omidenepag, (3) a salt of omidenepag, and (4) a salt of an ester of omidenepag.

When there are geometric isomers and/or optical isomers in omidenepag or an ester thereof or a salt thereof, those isomers are also included in the scope of the present invention.

When there is proton tautomerism in omidenepag or an ester thereof or a salt thereof, those tautomers (keto form and enol form) are also included in the scope of the present invention.

When there is crystal polymorphism and/or crystal polymorph group (crystal polymorph system) in omidenepag or an ester thereof or a salt thereof, those crystal polymorphs and/or crystal polymorph group (crystal polymorph system) are also included in the scope of the present invention. Here, the crystal polymorph group (crystal polymorph system) means a crystal form at each stage when the crystal form changes to various crystal forms depending on the conditions and/or states (incidentally, in this state, a formulated state is also included) of production, crystallization and preservation of these crystals, and/or the whole thereof.

In the therapeutic agent or the like of the present invention, omidenepag or an ester thereof or a salt thereof may take a form of a hydrate or a solvate.

In the therapeutic agent or the like of the present invention, a content of omidenepag or an ester thereof or a salt thereof is not particularly limited, which may vary depending on the administration form, and in the case of eye drops, a lower limit of the content of omidenepag or an ester thereof or a salt thereof is preferably 0.0003% (w/v), more preferably 0.001% (w/v), further preferably 0.0013% (w/v), and particularly preferably 0.0015% (w/v). Also, an upper limit of the above-mentioned content is preferably 0.03% (w/v), more preferably 0.01% (w/v), further preferably 0.005% (w/v), particularly preferably 0.003% (w/v), and especially preferably 0.0027% (w/v). In more detail, the above-mentioned content may be a range in which any of the above-mentioned lower limit and upper limit are combined, preferably 0.0003 to 0.03% (w/v), more preferably 0.001 to 0.01% (w/v), further preferably 0.001 to 0.005% (w/v), particularly preferably 0.001 to 0.003% (w/v), especially preferably 0.0013 to 0.003% (w/v), and peculiarly preferably 0.0015 to 0.0027% (w/v). More specifically, preferred examples include 0.0010% (w/v), 0.0011% (w/v), 0.0012% (w/v), 0.0013% (w/v), 0.0014% (w/v), 0.0015% (w/v), 0.0016% (w/v), 0.0017% (w/v), 0.0018% (w/v), 0.0019% (w/v), 0.0020% (w/v), 0.0021% (w/v), 0.0022% (w/v), 0.0023% (w/v), 0.0024% (w/v), 0.0025% (w/v), 0.0026% (w/v), 0.0027% (w/v), 0.0028% (w/v), 0.0029% (w/v), 0.0030% (w/v), 0.005% (w/v), 0.01% (w/v), 0.03% (w/v) and a range in which these amounts are set as the upper limit or the lower limit. Here, "% (w/v)" means a mass (g) of an active ingredient(s) (omidenepag or an ester thereof or a salt thereof, etc.) or an additive(s) (surfactant, etc.) contained in 100 mL of the drug. For example, 0.01% (w/v) omidenepag means that the content of omidenepag contained in 100 mL of the drug is 0.01 g.

Incidentally, when omidenepag or an ester thereof is in the form of a salt, a hydrate or a solvate, the contents of these omidenepag or an ester thereof or a salt thereof may be calculated based on any of a free form, a salt, a hydrate or a solvate of omidenepag or an ester thereof.

In the therapeutic agent or the like of the present invention, ripasudil is the compound (CAS registry number: 223645-67-8) represented by the following formula (3): 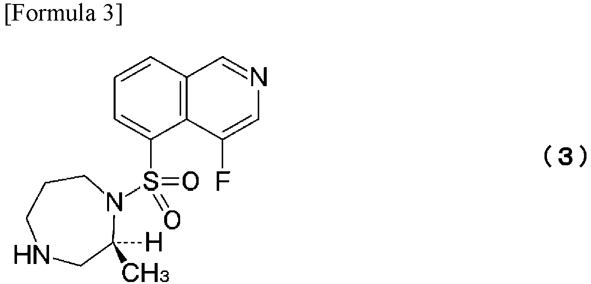 which is also referred to as (S)-(-)-1-(4-fluoro-5-isoquinolinesulfonyl)-2-methyl-1,4-homopiperazine. Since it has a Rho-associated coiled-coil containing protein kinase inhibitory action, and promotes drainage of aqueous humor from the main outflow passage via travecula-Schlemm's canal, it has been sold as a therapeutic agent for glaucoma and ocular hypertension (Glanatec (Registered Trademark) eye drops 0.4%).

In the therapeutic agent or the like of the present invention, the salt of ripasudil is not particularly limited as long as it is a pharmacologically acceptable salt. Specific examples include an inorganic acid salt such as hydrochloride, hydrobromide, hydroiodide, nitrate, sulfate or phosphate; an organic acid salt such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate or aspartate; a metal salt such as sodium salt, potassium salt, calcium salt or magnesium salt; an inorganic salt such as ammonium salt; or an organic amine salt such as triethylamine salt or guanidine salt, preferably hydrochloride, and further preferably monohydrochloride.

When there are geometric isomers and/or optical isomers in ripasudil or a salt thereof, those isomers are also included in the scope of the present invention.

When there is proton tautomerism in ripasudil or a salt thereof, those tautomers (keto form and enol form) are also included in the scope of the present invention.

When there is crystal polymorphism and/or crystal polymorph group (crystal polymorph system) in ripasudil or a salt thereof, those crystal polymorphs and/or crystal polymorph group (crystal polymorph system) are also included in the scope of the present invention. Here, the crystal polymorph group (crystal polymorph system) means a crystal form at each stage when the crystal form changes to various crystal forms depending on the conditions and/or states (incidentally, in this state, a formulated state is also included) of production, crystallization and preservation of these crystals, and/or the whole thereof.

In the therapeutic agent or the like of the present invention, ripasudil or a salt thereof may take a form of a hydrate or a solvate. As the salt and hydrate of ripasudil, ripasudil monohydrochloride dihydrate (CAS registry number; 887375-67-9) is most preferable. In the therapeutic agent or the like of the present invention, ripasudil or a salt thereof, or a hydrate or a solvate thereof is also simply referred to as "ripasudil".

In the therapeutic agent or the like of the present invention, a content of ripasudil or a salt thereof is not particularly limited, which may vary depending on the administration form, and in the case of eye drops, a lower limit of the content of ripasudil or a salt thereof is preferably 0.01% (w/v), more preferably 0.05% (w/v), further preferably 0.1% (w/v), and particularly preferably 0.2% (w/v). Also, an upper limit of the above-mentioned content is preferably 3% (w/v), more preferably 2% (w/v), further preferably 1% (w/v), and particularly preferably 0.6% (w/v). In more detail, the above-mentioned content may be a range in which any of the above-mentioned lower limit and upper limit are combined, and preferably 0.01 to 3% (w/v), more preferably 0.05 to 2% (w/v), further preferably 0.1 to 1% (w/v), particularly preferably 0.2 to 0.6% (w/v), and most preferably 0.4% (w/v).

Incidentally, when ripasudil is in the form of a salt, a hydrate or a solvate, the contents of these ripasudil or a salt thereof may be calculated based on any of a free form, a salt, a hydrate or a solvate of ripasudil.

In the therapeutic agent or the like of the present invention, netarsudil is the compound (CAS registry number: 1254032-66-0) represented by the following formula (4): 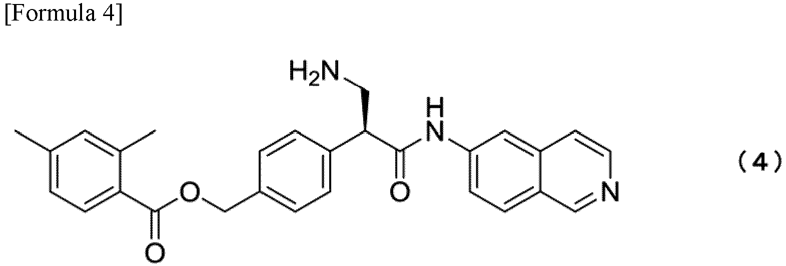 which is also referred to as [4-[(1S)-1-(aminomethyl)-2-(isoquinolin-6-ylamino)-2-oxoethyl]phenyl]methyl 2,4-dimethylbenzoate. Since it has a Rho-associated coiled-coil containing protein kinase inhibitory action and a norepinephrine transporter (NEP) inhibitory action, and exhibits an intraocular pressure lowering action, it has been sold as a therapeutic agent for glaucoma and ocular hypertension in the United States (RHOPRESSA (Registered Trademark) 0.02%).

In the therapeutic agent or the like of the present invention, the salt of netarsudil is not particularly limited as long as it is a pharmacologically acceptable salt. Specific examples include an inorganic acid salt such as hydrochloride, hydrobromide, hydroiodide, nitrate, sulfate or phosphate; an organic acid salt such as acetate, trifluoroacetate, benzoate, oxalate, malonate, succinate, maleate, fumarate, tartrate, citrate, mesylate (methanesulfonate), ethanesulfonate, trifluoromethanesulfonate, benzenesulfonate, p-toluenesulfonate, glutamate or aspartate; a metal salt such as sodium salt, potassium salt, calcium salt or magnesium salt; an inorganic salt such as ammonium salt; or an organic amine salt such as triethylamine salt or guanidine salt, preferably mesylate (methanesulfonate) or hydrochloride, and more preferably dimesylate (dimethanesulfonate) or dihydrochloride.

When there are geometric isomers and/or optical isomers in netarsudil or a salt thereof, those isomers are also included in the scope of the present invention.

When there is proton tautomerism in netarsudil or a salt thereof, those tautomers (keto form and enol form) are also included in the scope of the present invention.

When there is crystal polymorphism and/or crystal polymorph group (crystal polymorph system) in netarsudil or a salt thereof, those crystal polymorphs and/or crystal polymorph group (crystal polymorph system) are also included in the scope of the present invention. Here, the crystal polymorph group (crystal polymorph system) means a crystal form at each stage when the crystal form changes to various crystal forms depending on the conditions and/or states (incidentally, in this state, a formulated state is also included) of production, crystallization and preservation of these crystals, and/or the whole thereof.

In the therapeutic agent or the like of the present invention, netarsudil or a salt thereof may take a form of a hydrate or a solvate. As the salt and hydrate of netarsudil, netarsudil dimesylate (CAS registry number: 1422144-42-0) is most preferable. In the therapeutic agent or the like of the present invention, netarsudil or a salt thereof, or a hydrate or a solvate thereof is also simply referred to as "netarsudil".

In the therapeutic agent or the like of the present invention, a content of netarsudil or a salt thereof is not particularly limited, which may vary depending on the administration form, and in the case of eye drops, a lower limit of the content of netarsudil or a salt thereof to be contained in the drug of the present invention is preferably 0.001% (w/v), more preferably 0.003% (w/v), further preferably 0.005% (w/v), and particularly preferably 0.01% (w/v). Also, an upper limit of the above-mentioned content is preferably 0.2% (w/v), more preferably 0.1% (w/v), further preferably 0.06% (w/v), and particularly preferably 0.04% (w/v). In more detail, the above-mentioned content may be a range in which any of the above-mentioned lower limit and upper limit are combined, and preferably 0.001 to 0.2% (w/v), more preferably 0.003 to 0.1% (w/v), further preferably 0.005 to 0.06% (w/v), particularly preferably 0.01 to 0.04% (w/v), and most preferably 0.02% (w/v).

Incidentally, when netarsudil is in the form of a salt, a hydrate or a solvate, the contents of these netarsudil or a salt thereof may be calculated based on any of a free form, a salt, a hydrate or a solvate of netarsudil.

In the therapeutic agent or the like of the present invention, in addition to omidenepag or an ester thereof or a salt thereof, and one or more of the Rho-associated coiled-coil containing protein kinase inhibitors selected from the group consisting of ripasudil, netarsudil and a salt thereof, one or more of the other prophylactic or therapeutic agent(s) for glaucoma or ocular hypertension may be further used in combination. The other prophylactic or therapeutic agent(s) for glaucoma or ocular hypertension may be any drug as long as it has an intraocular pressure lowering action and is useful for the treatment for glaucoma, and there may be mentioned non-selective sympathomimetics, α₂ receptor agonists, α ₁ receptor blockers, β receptor blockers, parasympathomimetics, carbonic anhydrase inhibitors, prostaglandins and the like.

Specific examples of the non-selective sympathomimetics include dipivefrin, specific examples of the α₂ receptor agonists include brimonidine and apraclonidine, specific examples of the α₁ receptor blockers include bunazosin, specific examples of the β receptor blockers include timolol, befunolol, carteolol, nipradilol, betaxolol, levobunolol and metipranolol, specific examples of the parasympathomimetics include pilocarpine, specific examples of the carbonic anhydrase inhibitors include dorzolamide, brinzolamide and acetazolamide, and specific examples of the prostaglandins include isopropyl unoprostone, latanoprost, travoprost and bimatoprost. These include a form of a salt pharmaceutically acceptable as a medicine. Specific examples of the salt include an inorganic acid salt such as hydrochloride, hydrobromide, hydroiodide, nitrate, sulfate or phosphate; an organic acid salt such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate or aspartate; a metal salt such as sodium salt, potassium salt, calcium salt or magnesium salt; an inorganic salt such as ammonium salt; or an organic amine salt such as triethylamine salt or guanidine salt.

Further, the other prophylactic or therapeutic agent(s) for glaucoma or ocular hypertension may take a form of a hydrate or a solvate.

In the therapeutic agent or the like of the present invention, when it is used in combination with the other prophylactic or therapeutic agent(s) for glaucoma or ocular hypertension, a content thereof is not particularly limited, which may vary depending on a kind and an administration form of the prophylactic or therapeutic agent to be contained, and a preferred content in the case of eye drops is as follows.

The content of the non-selective sympathomimetics may vary depending on a kind of the drug, and in the case of dipivefrin, it is preferably 0.001 to 3% (w/v), more preferably 0.04 to 0.1% (w/v), and particularly preferably 0.04% (w/v) or 0.1% (w/v).

The content of the α₂ receptor agonists may vary depending on a kind of the drug, and in the case of brimonidine, it is preferably 0.01 to 5% (w/v), more preferably 0.1 to 0.5% (w/v), and particularly preferably 0.1% (w/v), 0.15% (w/v), 0.2% (w/v) or 0.5% (w/v). Also, in the case of apraclonidine, it is preferably 0.01 to 5% (w/v), more preferably 0.5 to 1% (w/v), and particularly preferably 0.5% (w/v).

The content of the α₁ receptor blockers may vary depending on a kind of the drug, and in the case of bunazosin, it is preferably 0.001 to 0.3% (w/v), more preferably 0.003 to 0.03% (w/v), and particularly preferably 0.01% (w/v).

The content of the β receptor blockers may vary depending on a kind of the drug, and in the case of timolol, it is preferably 0.01 to 5% (w/v), more preferably 0.1 to 0.5% (w/v), and particularly preferably 0.1% (w/v), 0.25% (w/v) or 0.5% (w/v). Also, in the case of befunolol, it is preferably 0.01 to 5% (w/v), more preferably 0.25 to 1% (w/v), and particularly preferably 0.25% (w/v), 0.5% (w/v) or 1% (w/v). In the case of carteolol, it is preferably 0.01 to 5% (w/v), more preferably 1 to 2% (w/v), and particularly preferably 1% (w/v) or 2% (w/v). In the case of nipradilol, it is preferably 0.01 to 5% (w/v), and particularly preferably 0.25% (w/v). In the case of betaxolol, it is preferably 0.01 to 5% (w/v), more preferably 0.25 to 0.5% (w/v), and particularly preferably 0.25% (w/v) or 0.5% (w/v). In the case of levobunolol, it is preferably 0.01 to 5% (w/v), more preferably 0.25 to 0.5% (w/v), and particularly preferably 0.25% (w/v) or 0.5% (w/v). In the case of metipranolol, it is preferably 0.01 to 5% (w/v), and particularly preferably 0.3% (w/v).

The content of the parasympathomimetics may vary depending on a kind of the drug, and in the case of pilocarpine, it is preferably 0.01 to 20% (w/v), more preferably 0.1 to 5% (w/v), and particularly preferably 0.5% (w/v), 1% (w/v), 2% (w/v), 3% (w/v) or 4% (w/v).

The content of the carbonic anhydrase inhibitors may vary depending on a kind of the drug, and in the case of dorzolamide, it is preferably 0.01 to 5% (w/v), more preferably 0.5 to 2% (w/v), and particularly preferably 0.5% (w/v), 1% (w/v) or 2% (w/v). Also, in the case of brinzolamide, it is preferably 0.01 to 5% (w/v), more preferably 0.1 to 2% (w/v), and particularly preferably 1% (w/v). Also, in the case of acetazolamide, it is preferably 0.01 to 5% (w/v), and more preferably 1 to 5% (w/v). Incidentally, when acetazolamide is orally administered, 250 to 1000 mg may be used as a daily dose.

The content of the prostaglandins may vary depending on a kind of the drug, and in the case of latanoprost, it is preferably 0.0001 to 5% (w/v), more preferably 0.0005 to 1% (w/v), further preferably 0.001 to 0.1% (w/v), and particularly preferably 0.005% (w/v). In the case of isopropyl unoprostone, it is preferably 0.001 to 5% (w/v), more preferably 0.01 to 1% (w/v), further preferably 0.12 to 0.15% (w/v), and particularly preferably 0.12% (w/v) or 0.15% (w/v). In the case of bimatoprost, it is preferably 0.0001 to 5% (w/v), more preferably 0.001 to 1% (w/v), further preferably 0.01 to 0.03% (w/v), and particularly preferably 0.01% (w/v) or 0.03% (w/v). In the case of travoprost, it is preferably 0.0001 to 5% (w/v), more preferably 0.001 to 1% (w/v), and particularly preferably 0.004% (w/v).

Incidentally, when the other prophylactic or therapeutic agent(s) for glaucoma or ocular hypertension is in the form of a salt, a hydrate or a solvate, the content of the other prophylactic or therapeutic agent(s) for glaucoma or ocular hypertension may be calculated based on any of a free form, a salt, a hydrate or a solvate of the other prophylactic or therapeutic agent(s) for glaucoma or ocular hypertension.

The therapeutic agent or the like of the present invention is characterized in that omidenepag or an ester thereof or a salt thereof, and one or more of the Rho-associated coiled-coil containing protein kinase inhibitors selected from the group consisting of ripasudil, netarsudil and a salt thereof are administered in combination whereby glaucoma or ocular hypertension is to be prevented or treated. As the glaucoma in the therapeutic agent or the like of the present invention, primary open-angle glaucoma, secondary open-angle glaucoma, normal tension glaucoma, hypersecretion glaucoma, primary angle-closure glaucoma, secondary angle-closure glaucoma, plateau iris glaucoma, combined-mechanism glaucoma, developmental glaucoma, steroid induced glaucoma, exfoliation glaucoma, amyloid glaucoma, neovascular glaucoma, malignant glaucoma, capsular glaucoma of the lens, plateau iris syndrome and the like are exemplified.

In the therapeutic agent or the like of the present invention, as for the dosage form, a formulation comprising omidenepag or an ester thereof or a salt thereof, and a separate formulation comprising one or more of the Rho-associated coiled-coil containing protein kinase inhibitors selected from the group consisting of ripasudil, netarsudil and a salt thereof may be administered (concomitant administration), or a single formulation (combination drug) comprising omidenepag or an ester thereof or a salt thereof, and one or more of the Rho-associated coiled-coil containing protein kinase inhibitors selected from the group consisting of ripasudil, netarsudil and a salt thereof may be administered. Also, when it contains, in addition to omidenepag or an ester thereof or a salt thereof, and one or more of the Rho-associated coiled-coil containing protein kinase inhibitors selected from the group consisting of ripasudil, netarsudil and a salt thereof, further one or more of the other prophylactic or therapeutic agent(s) for glaucoma or ocular hypertension, then, omidenepag or an ester thereof or a salt thereof, one or more of the Rho-associated coiled-coil containing protein kinase inhibitors selected from the group consisting of ripasudil, netarsudil and a salt thereof, and the other prophylactic or therapeutic agent(s) for glaucoma or ocular hypertension may be administered concomitantly, a combination drug comprising optional component(s) of these and the remaining component(s) may be administered concomitantly, or a combination drug comprising all the components may be administered.

The therapeutic agent or the like of the present invention may be administered orally or parenterally, no particular technique is required for formulation thereof, and a formulation can be prepared by using a commonly used technique. As dosage forms, there may be mentioned eye drops, eye ointments, injections, tablets, capsules, granules, powders and the like, and eye drops or eye ointments are preferred.

When omidenepag or an ester thereof or a salt thereof, one or more of the Rho-associated coiled-coil containing protein kinase inhibitors selected from the group consisting of ripasudil, netarsudil and a salt thereof, and the other prophylactic or therapeutic agent(s) for glaucoma or ocular hypertension are separately formulated, formulations can be each prepared according to the known method. For example, a formulation of omidenepag or an ester thereof or a salt thereof can be prepared with reference to Preparation examples described in WO 2009/113600 or WO 2010/113957. As a formulation of the Rho-associated coiled-coil containing protein kinase inhibitor or the other prophylactic or therapeutic agent(s) for glaucoma or ocular hypertension, formulations already commercially available such as ripasudil, netarsudil, dipivefrin, brimonidine , apraclonidine, bunazosin, timolol, befunolol, carteolol, nipradilol, betaxolol, levobunolol, metipranolol, pilocarpine, dorzolamide, brinzolamide, acetazolamide, isopropyl unoprostone, latanoprost, travoprost, bimatoprost, Cosopt (Registered Trademark) combination eye drops, Xalacom (Registered Trademark) combination eye drops, DuoTrav (Registered Trademark) combination eye drops and the like or a substance(s) corresponding to these may be also used.

Also, when one formulation containing the respective components is to be prepared, it can be prepared according to a known method.

In the case of preparing eye drops, omidenepag or an ester thereof or a salt thereof, and one or more of the Rho-associated coiled-coil containing protein kinase inhibitors selected from the group consisting of ripasudil, netarsudil and a salt thereof are added to purified water, a buffer solution or the like, and stirred, and then, a pH of the mixture is adjusted with a pH adjusting agent to prepare a desired eye drop. In addition, if necessary, an additive(s) commonly used in eye drops may be used, and as the additives, there may be mentioned an isotonic agent, a buffering agent, a surfactant, a stabilizer, a preservative, and the like. As the isotonic agent, there may be mentioned sodium chloride, glycerin, and the like, as the buffering agent, there may be mentioned sodium phosphate, sodium acetate, boric acid, borax, citric acid, sodium citrate, and the like, as the surfactant, there may be mentioned polyoxyethylene sorbitan monooleate, polyoxyl stearate, polyoxyethylene castor oil, polyoxyethylene hydrogenated castor oil, and the like, as the stabilizer, there may be mentioned sodium citrate, sodium edetate, and the like, and as the preservative, there may be mentioned benzalkonium chloride, paraben, and the like.

A pH of the eye drops may be within the range which is allowable for ophthalmic formulations, it is preferably in the range of pH 4 to 8, and more preferably in the range of pH 5 to 7.

In the case of preparing eye ointments, it can be prepared by using a commonly used base, and as the base, there may be mentioned white petrolatum, liquid paraffin, and the like.

In the case of preparing oral formulations such as tablets, capsules, granules, powders, and the like, it can be prepared by adding a bulking agent, a lubricant, a binder, a disintegrating agent, a coating agent, a film agent, and the like, as necessary. As the bulking agent, there may be mentioned lactose, crystalline cellulose, starch, vegetable oil, and the like, as the lubricant, there may be mentioned magnesium stearate, talc, and the like, as the binder, there may be mentioned hydroxypropyl cellulose, polyvinylpyrrolidone, and the like, as the disintegrating agent, there may be mentioned carboxymethylcellulose calcium, low-substituted hydroxypropylmethyl cellulose, and the like, as the coating agent, there may be mentioned hydroxypropyl methylcellulose, macrogol, silicone resin, and the like, and as the film agent, there may be mentioned a gelatin film, and the like.

An administration method of the therapeutic agent or the like of the present invention can be appropriately changed depending on the dosage form, the severity of symptoms of a patient to be administered to, the age, the body weight, the administration route, the judgment of a doctor, and the like, and in the case of a combination drug comprising omidenepag or an ester thereof or a salt thereof, and one or more of the Rho-associated coiled-coil containing protein kinase inhibitors selected from the group consisting of ripasudil, netarsudil and a salt thereof, it may be administered 1 to 5 times a day, preferably once or twice a day, and most preferably once a day. When a formulation comprising omidenepag or an ester thereof or a salt thereof, and a formulation comprising one or more of the Rho-associated coiled-coil containing protein kinase inhibitors selected from the group consisting of ripasudil, netarsudil and a salt thereof are administered concomitantly, each formulation may be administered at different times or simultaneously 1 to 3 times a day, preferably once or twice a day, and most preferably once a day. Incidentally, in the concomitant administration, when the formulations are administered at different times, the order of administering the formulations is not limited, and after one formulation is administered, the other formulation may be administered within 12 hours, preferably within 6 hours, more preferably within 1 hour, further preferably within 30 minutes, particularly preferably within 5 minutes, and most preferably promptly. In the above-mentioned administration method, in the case of eye drop administration, it is preferable to administer 1 to 3 drops per once, more preferably to administer 1 or 2 drops, and most preferably to administer 1 drop.

The detailed description of the above-mentioned therapeutic agent or the like of the present invention is also applied to the prophylactic or therapeutic agent for glaucoma or ocular hypertension of the present invention, comprising omidenepag or an ester thereof or a salt thereof, which is characterized by being used concomitantly with one or more of the Rho-associated coiled-coil containing protein kinase inhibitors selected from the group consisting of ripasudil, netarsudil and a salt thereof. The detailed description of the above-mentioned therapeutic agent or the like of the present invention is also applied to the intraocular pressure-lowering agent of the present invention, which is characterized in that omidenepag or an ester thereof or a salt thereof, and one or more of the Rho-associated coiled-coil containing protein kinase inhibitors selected from the group consisting of ripasudil, netarsudil and a salt thereof are combined. The detailed description of the above-mentioned therapeutic agent or the like of the present invention is also applied to the intraocular pressure-lowering agent of the present invention, comprising omidenepag or an ester thereof or a salt thereof, which is characterized by being used concomitantly with one or more of the Rho-associated coiled-coil containing protein kinase inhibitors selected from the group consisting of ripasudil, netarsudil and a salt thereof.

Also, detailed description of the above-mentioned therapeutic agent or the like of the present invention is also applied to the embodiment of the present invention mentioned below.

One embodiment of the present invention is a composition for the prophylaxis or treatment for glaucoma or ocular hypertension comprising omidenepag or an ester thereof or a salt thereof, which is characterized by being administered in combination with one or more of the Rho-associated coiled-coil containing protein kinase inhibitors selected from the group consisting of ripasudil, netarsudil and a salt thereof.

One embodiment of the present invention is a prophylactic or therapeutic method for glaucoma or ocular hypertension comprising: administering a therapeutically effective amount of omidenepag or an ester thereof or a salt thereof, and a therapeutically effective amount of one or more of the Rho-associated coiled-coil containing protein kinase inhibitors selected from the group consisting of ripasudil, netarsudil and a salt thereof in combination to a subject in need thereof.

One embodiment of the present invention is use of a combination of omidenepag or an ester thereof or a salt thereof, and one or more of the Rho-associated coiled-coil containing protein kinase inhibitors selected from the group consisting of ripasudil, netarsudil and a salt thereof for manufacturing a medicament for the prophylaxis or treatment for glaucoma or ocular hypertension

One embodiment of the present invention is use of omidenepag or an ester thereof or a salt thereof for manufacturing a medicament for the prophylaxis or treatment for glaucoma or ocular hypertension characterized by being used concomitantly with one or more of the Rho-associated coiled-coil containing protein kinase inhibitors selected from the group consisting of ripasudil, netarsudil and a salt thereof.

One embodiment of the present invention is omidenepag or an ester thereof or a salt thereof for use in the prophylaxis or treatment for glaucoma or ocular hypertension, which is characterized by being used concomitantly with one or more of the Rho-associated coiled-coil containing protein kinase inhibitors selected from the group consisting of ripasudil, netarsudil and a salt thereof.

One embodiment of the present invention is a combination of omidenepag or an ester thereof or a salt thereof, and one or more of the Rho-associated coiled-coil containing protein kinase inhibitors selected from the group consisting of ripasudil, netarsudil and a salt thereof for use in the prophylaxis or treatment for glaucoma or ocular hypertension.

One embodiment of the present invention is a composition for lowering an intraocular pressure comprising omidenepag or an ester thereof or a salt thereof, which is characterized by being administered in combination with one or more of the Rho-associated coiled-coil containing protein kinase inhibitors selected from the group consisting of ripasudil, netarsudil and a salt thereof.

One embodiment of the present invention is a method for lowering an intraocular pressure comprising: administering a therapeutically effective amount of omidenepag or an ester thereof or a salt thereof, and a therapeutically effective amount of one or more of the Rho-associated coiled-coil containing protein kinase inhibitors selected from the group consisting of ripasudil, netarsudil and a salt thereof to a subject in need thereof.

One embodiment of the present invention is use of a combination of omidenepag or an ester thereof or a salt thereof, and one or more of the Rho-associated coiled-coil containing protein kinase inhibitors selected from the group consisting of ripasudil, netarsudil and a salt thereof for manufacturing a medicament for lowering an intraocular pressure.

One embodiment of the present invention is use of omidenepag or an ester thereof or a salt thereof for manufacturing a medicament for lowering an intraocular pressure characterized by being used concomitantly with one or more of the Rho-associated coiled-coil containing protein kinase inhibitors selected from the group consisting of ripasudil, netarsudil and a salt thereof.

One embodiment of the present invention is omidenepag or an ester thereof or a salt thereof for use in lowering an intraocular pressure, which is characterized by being used concomitantly with one or more of the Rho-associated coiled-coil containing protein kinase inhibitors selected from the group consisting of ripasudil, netarsudil and a salt thereof.

One embodiment of the present invention is a combination of omidenepag or an ester thereof or a salt thereof, and one or more of the Rho-associated coiled-coil containing protein kinase inhibitors selected from the group consisting of ripasudil, netarsudil and a salt thereof for use in lowering an intraocular pressure.

### EXAMPLES

In the following, Formulation examples and pharmacological tests are shown, but these are for better understanding of the present invention and do not limit the scope of the present invention.

### [Formulation examples]

Specific Formulation examples of eye drops and eye ointments comprising omidenepag or an ester thereof or a salt thereof, and one or more of the Rho-associated coiled-coil containing protein kinase inhibitors selected from the group consisting of ripasudil, netarsudil and a salt thereof according to the present invention are shown below.

### [Preparation example 1]

Eye drop (in 100 mL)

| | |
|---|---|
| Omidenepag isopropyl | 0.002g |
| Ripasudil monohydrochloride dihydrate | 0.4896g |
| Sodium dihydrogen phosphate | 0.15g |
| Glycerin | Appropriate amount |
| Polyoxyl 35 castor oil | 1.7g |
| Sodium edetate | 0.05g |
| Benzalkonium chloride | 0.005g |
| Diluted hydrochloric acid | Appropriate amount |
| Sodium hydroxide | Appropriate amount |
| Purified water | Appropriate amount |

### [Preparation example 2]

Eye drop (in 100 mL)

| | |
|---|---|
| Omidenepag isopropyl | 0.002g |
| Netarsudil | 0.02g |
| Sodium dihydrogen phosphate | 0.15g |
| Glycerin | Appropriate amount |
| Polyoxyl 35 castor oil | 1.7g |
| Sodium edetate | 0.05g |
| Benzalkonium chloride | 0.005g |
| Diluted hydrochloric acid | Appropriate amount |
| Sodium hydroxide | Appropriate amount |
| Purified water | Appropriate amount |

### [Preparation example 3]

Eye drop (in 100 mL)

| | |
|---|---|
| Omidenepag isopropyl | 0.002g |
| Netarsudil dimesylate | 0.02g |
| Sodium dihydrogen phosphate | 0.15g |
| Glycerin | Appropriate amount |
| Polyoxyl 35 castor oil | 1.7g |
| Sodium edetate | 0.05g |
| Benzalkonium chloride | 0.005g |

| | |
|---|---|
| Diluted hydrochloric acid | Appropriate amount |
| Sodium hydroxide | Appropriate amount |
| Purified water | Appropriate amount |

### [Preparation example 4]

Eye ointment (in 100 g)

| | |
|---|---|
| Omidenepag isopropyl | 0.01g |
| Ripasudil monohydroxide dihydrate | 0.5g |
| Liquid paraffin | 10.0g |
| White petrolatum | Appropriate amount |

### [Preparation example 5]

Eye ointment (in 100 g)

| | |
|---|---|
| Omidenepag isopropyl | 0.01g |
| Netarsudil | 0.5g |
| Liquid paraffin | 10.0g |
| White petrolatum | Appropriate amount |

In the above-mentioned prescription, by changing the kind and the amount of omidenepag or an ester thereof or a salt thereof, one or more of the Rho-associated coiled-coil containing protein kinase inhibitors selected from the group consisting of ripasudil, netarsudil and a salt thereof, and the additive(s), eye drops and eye ointments having a desired combination and desired concentration(s) can be prepared.

### [Pharmacological test]

### [Example 1]

In order to examine usefulness of the combination of omidenepag or an ester thereof or a salt thereof and ripasudil, the effect of lowering an intraocular pressure when omidenepag isopropyl and ripasudil were administered concomitantly to experimental animals (normal pressure monkeys) was investigated.

### (Preparation of compound solutions to be tested)

### (1) Preparation of base

To purified water were added polyoxyl 35 castor oil, glycerin, sodium citrate, sodium edetate and benzalkonium chloride to dissolve them, and after adjusting pH, purified water was added to adjust the whole amount.

### (2) Preparation of omidenepag isopropyl solution

To purified water were added omidenepag isopropyl, polyoxyl 35 castor oil, glycerin, sodium citrate, sodium edetate and benzalkonium chloride to dissolve them, and after adjusting pH, purified water was added to adjust the whole amount to prepare a 0.0006 w/v% omidenepag isopropyl solution.

### (3) Preparation of physiological saline solution

Commercially available physiological saline solution (trade name: Otsuka Normal Saline, obtained from Otsuka Pharmaceutical Factory, Inc.) was used as it was.

### (4) Preparation of ripasudil solution

Commercially available ripasudil eye drop was used as it was.

### (Test method)

An effect of lowering an intraocular pressure when omidenepag isopropyl and ripasudil were administered concomitantly was investigated. As a comparative subject, an effect of lowering an intraocular pressure when omidenepag isopropyl or ripasudil was administered alone was also investigated. As a control, the base and the physiological saline solution were administered.

### (Drugs and animals used in the test)

Omidenepag isopropyl solution: 0.0006w/v% omidenepag isopropyl solution (volume of eye dropped: 20 µL)
Ripasudil solution: ripasudil eye drop (trade name: Glanatec (Registered Trademark) eye drops 0.4%, volume of eye dropped: 20 µL)
Experimental animal: cynomolgus monkey (sex: male, 7 monkeys per a group)

### (Administration method and measurement method)

### [1] Concomitant administration of omidenepag isopropyl and ripasudil

(1) A drop of 0.4% oxybuprocaine hydrochloride eye drop (trade name: Benoxil (Registered Trademark) eye drops 0.4%) was applied to one eye of an experimental animal and local anesthesia was conducted.
(2) Immediately before administration of a compound solution to be tested, an intraocular pressure was measured and the value was made an initial intraocular pressure.
(3) The omidenepag isopropyl solution was applied to one eye of the experimental animal (the contralateral eye was untreated). After a short time (after about 5 minutes), the ripasudil solution was applied to the same eye.
(4) After 2 hours, 4 hours, 6 hours, 8 hours and 24 hours from applying the omidenepag isopropyl solution to the eye, one drop of 0.4% oxybuprocaine hydrochloride eye drop was applied to the eye to be measured for the intraocular pressure respectively, and after local anesthesia, the intraocular pressure was measured. Also, the intraocular pressure was measured each three times, and the average value was shown in the results.

### [2] Single administration of omidenepag isopropyl

The test was carried out in the same manner as the above-mentioned concomitant administration test except for changing the ripasudil solution to the physiological saline solution.

### [3] Single administration of ripasudil

The test was carried out in the same manner as the above-mentioned concomitant administration test except for changing the omidenepag isopropyl solution to the base.

### [4] Control

The test was carried out in the same manner as the above-mentioned concomitant administration test except for changing the omidenepag isopropyl solution to the base and changing the ripasudil solution to the physiological saline solution.

### (Results and consideration)

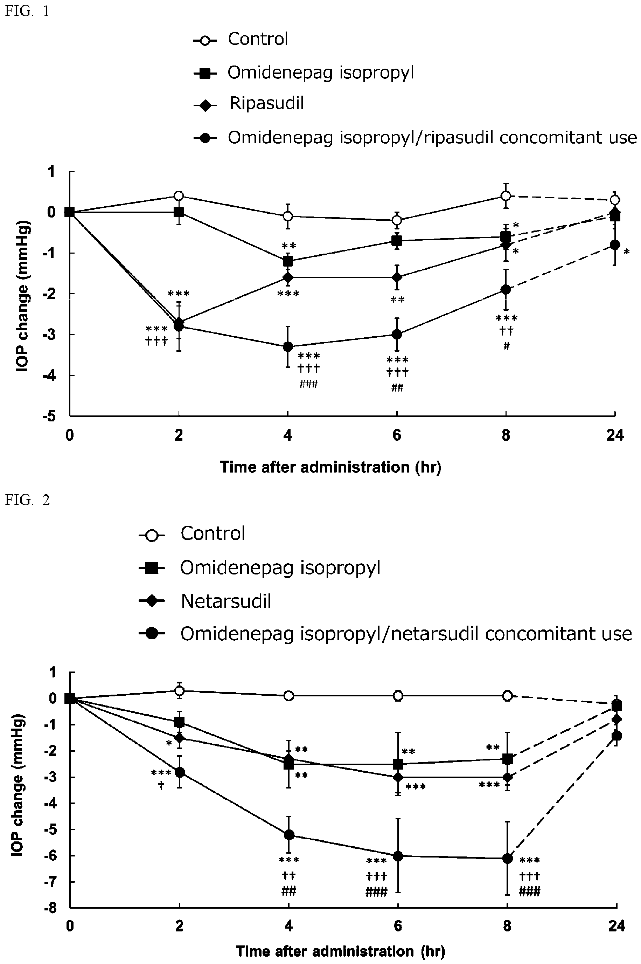

The changes in intraocular pressure values with the lapse of time for each administered group are shown in FIG. 1 and Table 1. The changes in the intraocular pressure values are shown by an average value ± SEM of the difference from the value (0 hour) before administration of seven monkeys in each group with regard to each measurement time point of each individual. Here, the significance was evaluated by the Dunnett-Hsu test, and the significance level with respect to the control group was shown as *: p<0.05, **: p<0.01, and ***: p<0.001, the significance level with respect to the omidenepag isopropyl administered group was shown as ††: p<0.01, and †††: p<0.001, and the significance level with respect to the ripasudil administered group was shown as #: p<0.05, ##: p<0.01, and ###: p<0.001.

**[Table 1]**

| Time after dropping | 2 | 4 | 6 | 8 | 24 |
|---|---|---|---|---|---|
| Control | 0.4 | -0.1 | -0.2 | 0.4 | 0.3 |
| Omidenepag isopropyl | 0.0 | -1.2 | -0.7 | -0.6 | -0.1 |
| Ripasudil | -2.7 | -1.6 | -1.6 | -0.8 | 0.0 |
| Omidenepag isopropyl/ ripasudil concomitant use | -2.8 | -3.3 | -3.0 | -1.9 | -0.8 |

As clearly seen from FIG. 1 and Table 1, the concomitantly administered group of omidenepag isopropyl and ripasudil showed more excellent intraocular pressure lowering action and sustained effect of the action than the single drug administered group, that is, the omidenepag isopropyl administered group and the ripasudil administered group. At all measurement times, the amounts of change in the intraocular pressure values for the concomitantly administered group of omidenepag isopropyl and ripasudil was larger than the sum of the amounts of change in the intraocular pressure values for the omidenepag isopropyl administered group and for the ripasudil administered group, and the synergistic effect of the intraocular pressure lowering action was confirmed.

From the above, it was found that by combining omidenepag or an ester thereof or a salt thereof with ripasudil, a synergistic effect of an intraocular pressure lowering action and a sustained effect of the action can be obtained.

### [Example 2]

In order to examine usefulness of the combination of omidenepag or an ester thereof or a salt thereof and netarsudil, the effect of lowering an intraocular pressure when omidenepag isopropyl and netarsudil were administered concomitantly to experimental animals (normal pressure monkeys) was investigated.

### (Preparation of compound solutions to be tested)

### (1) Preparation of base of omidenepag isopropyl solution

### (Preparation of compound solution to be tested)

To purified water were added polyoxyl 35 castor oil, glycerin, sodium citrate, sodium edetate and benzalkonium chloride to dissolve them, and after adjusting pH, purified water was added to adjust the whole amount.

### (2) Preparation of omidenepag isopropyl solution

To purified water were added omidenepag isopropyl, polyoxyl 35 castor oil, glycerin, sodium citrate, sodium edetate and benzalkonium chloride to dissolve them, and after adjusting pH, purified water was added to adjust the whole amount to prepare a 0.0006 w/v% omidenepag isopropyl solution.

### (3) Preparation of netarsudil solution

Dimesylate of netarsudil was dissolved in a physiological saline solution containing a solubilizing agent, and then, a netarsudil solution having a desired concentration was prepared by using a commonly used method.

### (Test method)

An effect of lowering an intraocular pressure when omidenepag isopropyl and netarsudil were administered concomitantly was investigated. As a comparative subject, an effect of lowering an intraocular pressure when omidenepag isopropyl or netarsudil was administered alone was also investigated. As a control, the base of the omidenepag isopropyl solution and the base of the netarsudil solution were administered.

### (Drugs and animals used in the test)

Omidenepag isopropyl solution: 0.0006w/v% omidenepag isopropyl solution (volume of eye dropped: 20 µL)
Netarsudil solution: 0.01 w/v% netarsudil solution (volume of eye dropped: 20 µL)
Experimental animal: cynomolgus monkey (sex: male, 8 monkeys per a group)

### (Administration method and measurement method)

### [1] Contamitant administration of omidenepag isopropyl and netarsudil

(1) A drop of 0.4% oxybuprocaine hydrochloride eye drop (trade name: Benoxil (Registered Trademark) eye drops 0.4%) was applied to one eye of an experimental animal and local anesthesia was conducted.
(2) Immediately before administration of a compound solution to be tested, an intraocular pressure was measured and the value was made an initial intraocular pressure.
(3) The omidenepag isopropyl solution was applied to one eye of the experimental animal (the contralateral eye was untreated). After a short time (after about 5 minutes), the netarsudil solution was applied to the same eye.
(4) After 2 hours, 4 hours, 6 hours, 8 hours and 24 hours from applying the omidenepag isopropyl solution to the eye, one drop of 0.4% oxybuprocaine hydrochloride eye drop was applied to the eye to be measured for the intraocular pressure respectively, and after local anesthesia, the intraocular pressure was measured. Also, the intraocular pressure was measured each three times, and the average value was shown in the results.

### [2] Single administration of omidenepag isopropyl

The test was carried out in the same manner as the above-mentioned concomitant administration test except for changing the netarsudil solution to the base of the netarsudil solution.

### [3] Single administration of netarsudil

The test was carried out in the same manner as the above-mentioned concomitant administration test except for changing the omidenepag isopropyl solution to the base of the omidenepag isopropyl solution.

### [4] Control

The test was carried out in the same manner as the above-mentioned concomitant administration test except for changing the omidenepag isopropyl solution to the base of the omidenepag isopropyl solution and changing the netarsudil solution to the base of the netarsudil solution.

### (Results and consideration)

The changes in intraocular pressure values with the lapse of time for each administered group are shown in FIG. 2 and Table 2. The changes in the intraocular pressure values are shown by an average value ± SEM of the difference from the value (0 hour) before administration of eight monkeys in each group with regard to each measurement time point of each individual. Here, the significance was evaluated by the Dunnett-Hsu test, and the significance level with respect to the control group was shown as *: p<0.05, **: p<0.01, and ***: p<0.001, the significance level with respect to the omidenepag isopropyl administered group was shown as †: p<0.05,††: p<0.01, and †††: p<0.001, and the significance level with respect to the netarsudil administered group was shown as ##: p<0.01, and ###: p<0.001.

**[Table 2]**

| Time after dropping | 2 | 4 | 6 | 8 | 24 |
|---|---|---|---|---|---|
| Control | 0.3 | 0.1 | 0.1 | 0.1 | -0.2 |
| Omidenepag isopropyl | -0.9 | -2.5 | -2.5 | -2.3 | -0.3 |
| Netarsudil | -1.5 | -2.3 | -3.0 | -3.0 | -0.8 |
| Omidenepag isopropyl/ netarsudil concomitant use | -2.8 | -5.2 | -6.0 | -6.1 | -1.4 |

As clearly seen from FIG. 2 and Table 2, the concomitantly administered group of omidenepag isopropyl and netarsudil showed excellent intraocular pressure lowering action and sustained effect of the action than the single drug administered group, that is, the omidenepag isopropyl administered group and the netarsudil administered group. At all measurement times, the amounts of change in the intraocular pressure values for the concomitantly administered group of omidenepag isopropyl and netarsudil was larger than the sum of the amounts of change in the intraocular pressure values for the omidenepag isopropyl administered group and for the netarsudil administered group, and the synergistic effect of the intraocular pressure lowering action was confirmed.

From the above, it was found that by combining omidenepag or an ester thereof or a salt thereof with netarsudil, a synergistic effect of an intraocular pressure lowering action and a sustained effect of the action can be obtained.

### INDUSTRIAL APPLICABILITY

When omidenepag or an ester thereof or a salt thereof, and one or more of the Rho-associated coiled-coil containing protein kinase inhibitors selected from the group consisting of ripasudil, netarsudil and a salt thereof are combined and administered to an eye, an intraocular pressure lowering action is enhanced. Therefore, the present invention is useful as a prophylactic or therapeutic agent for glaucoma or ocular hypertension.

## Claims

1. A prophylactic or therapeutic agent for glaucoma or ocular hypertension, which is **characterized in that** omidenepag or an ester thereof or a salt thereof, and one or more of the Rho-associated coiled-coil containing protein kinase inhibitors selected from the group consisting of ripasudil, netarsudil and a salt thereof are administered in combination.

2. The prophylactic or therapeutic agent according to Claim 1, which is a combination drug comprising omidenepag or an ester thereof or a salt thereof, and one or more of the Rho-associated coiled-coil containing protein kinase inhibitors selected from the group consisting of ripasudil, netarsudil and a salt thereof.

3. The prophylactic or therapeutic agent according to Claim 1, wherein omidenepag or an ester thereof or a salt thereof, and one or more of the Rho-associated coiled-coil containing protein kinase inhibitors selected from the group consisting of ripasudil, netarsudil and a salt thereof are administered at different times or simultaneously.

4. A prophylactic or therapeutic agent for glaucoma or ocular hypertension comprising omidenepag or an ester thereof or a salt thereof, which is **characterized by** being used concomitantly with one or more of the Rho-associated coiled-coil containing protein kinase inhibitors selected from the group consisting of ripasudil, netarsudil and a salt thereof.

5. The prophylactic or therapeutic agent according to Claim 4, which is administered at different time from or simultaneously with one or more of the Rho-associated coiled-coil containing protein kinase inhibitors selected from the group consisting of ripasudil, netarsudil and a salt thereof.

6. The prophylactic or therapeutic agent according to any one of Claims 1 to 5, wherein omidenepag or an ester thereof or a salt thereof is omidenepag isopropyl.

7. The prophylactic or therapeutic agent according to any one of Claims 1 to 6, wherein the Rho-associated coiled-coil containing protein kinase inhibitor is ripasudil monohydrochloride dihydrate.

8. The prophylactic or therapeutic agent according to any one of Claims 1 to 6, wherein the Rho-associated coiled-coil containing protein kinase inhibitor is dimesylate or dihydrochloride of netarsudil.
